# EUROPEAN PATENT SPECIFICATION

(11) **EP 3 866 935 B1**
(45) Date of publication and mention of the grant of the patent: **10.08.2022**
(21) Application number: 19786787.2
(22) Date of filing: 14.10.2019
(51) Int. Cl.: A61Q 15/00, A61K 8/34, A61K 8/36, A61K 8/86

(54) **METHOD OF REDUCING PERSPIRATION**
VERFAHREN ZUR REDUZIERUNG VON PERSPIRATION
PROCÉDÉ DE RÉDUCTION DE LA TRANSPIRATION

(30) Priority: 18.10.2018 EP 18201196
(43) Date of publication of application: 25.08.2021
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: COURTOIS, Jean-Philippe, Andre, Roger, Wirral Merseyside CH63 3JW (GB); MARTINDALE, Daniel, Albert, John, Wirral Merseyside CH63 3JW (GB); WEDDELL, Iain, Andrew, Wirral Merseyside CH63 3JW (GB)
(74) Representative: Whaley, Christopher
(86) International application number: PCT/EP2019/077828
(87) International publication number: WO 2020/078931

(56) References cited:
- EP-A1- 0 550 960
- WO-A1-2017/182358
- DATABASE GNPD [Online] MINTEL; 10 August 2018 (2018-08-10), anonymous: "Deo Stick", XP055581211, retrieved from www.gnpd.com Database accession no. 5870363

## Description

### Field of Invention

This invention relates to the field of cosmetic products, in particular antiperspirant products and usage. This invention also relates to methods of reducing perspiration upon the surface of the human body.

### Background

Cosmetic compositions used for reducing perspiration often comprise an astringent metal salt, such as aluminium or zirconium salt. Such salts are perceived as harsh or irritating by some consumers and there is a need for alternative means of achieving sweat reduction.

The search for "non-AI" antiperspirant agents has led down numerous avenues. Some approaches have involved reducing sweating at source, i.e. the secretory coil of the sweat glands; however, those more closely related to the present invention have focussed on blocking or reducing perspiration from the upper parts of the sweat glands.

EP 2,442,779 B1 (Unilever, 2013) discloses the use of lamellar phase stabilised oil-in-water emulsions as antiperspirant agents.

WO 95/027473 (Gillette, 1995) discloses the use of selected film-forming polymers comprising pendant quaternary nitrogen groups as antiperspirant agents. There are other publications also disclosing the use of film-forming polymers as antiperspirant agents.

EP 550,960 A1 (Unilever, 1992) discloses the use as an antiperspirant active of an amphiphilic material which forms, upon contact with perspiration, a water-insoluble liquid crystal phase of greater than one dimensional periodicity.

The present invention utilises C18-C22 fatty acid soaps as antiperspirant agents. Such agents have been widely used in cosmetic compositions, including deodorant compositions; however, their antiperspirant properties have not been previously recognised.

A C18-C22 fatty acid soap widely used as a structurant in deodorant stick compositions is sodium stearate. This component is used in numerous commercial deodorant sticks, including deodorant sticks by Colgate ("Speed Stick 24/7"), Dial Corp. ("Right Guard") and P&G ("Old Spice"). None of these products, however, are advertised as antiperspirants.

Patent disclosures of the use of sodium stearate as a structurant in cosmetic compositions are numerous; however, none of these disclosures indicate or suggest that the sodium stearate is an antiperspirant agent.

### Summary of Invention

In a first aspect of the present invention, there is provided the use of a saturated C18-C22 fatty acid soap as an antiperspirant agent or sweat reducing agent.

The first aspect of the present invention may alternative be expressed as the use of a saturated C18-C22 fatty acid soap to give an antiperspirancy benefit, i.e. a reduction in perspiration.

In a second aspect of the present invention, there is provided the use for reducing perspiration of a composition comprising a saturated C18-C22 fatty acid soap and excluding any astringent aluminium or zirconium metal salt.

The use claimed in the present invention is cosmetic, i.e. non-therapeutic, use.

### Detailed Description of the Invention

Herein, antiperspirant agent' should be understood to mean a substance that by itself has the effect of reducing or limiting human perspiration.

'Free from aluminium and/or zirconium salt' or 'in absence of aluminium and/or zirconium salt' refers to a composition containing less than 1%, preferably less than 0.1% and more preferably, containing zero percent of aluminium and/or zirconium salt.

Herein, any feature of one aspect of the present invention may be utilised in any other aspect of the invention. Any feature described as 'preferred' should be understood to be particularly preferred in combination with a further preferred feature or features.

Herein, the word 'comprising' is intended to mean 'including' but not necessarily consisting of' or composed of'. In other words, the listed steps or options need not be exhaustive.

Herein, all percentages, parts and ratios are by weight unless otherwise indicated.

Except in the operating and comparative examples, or where otherwise explicitly indicated, all numbers in this description indicating amounts of material or conditions of reaction, physical properties or materials and/or use are to be understood as modified by the word 'about'.

Numerical ranges expressed in the format 'from x to y' are understood to include x and y.

The methods and uses of the present invention are typically achieved by the topical application of one or more cosmetic compositions comprising the fatty acid soap. The methods and uses lead to reduction in perspiration or sweating, which can enhance an individual's comfort, appearance, and/or confidence.

The topical application typically employed in the use of the present invention is to the human skin, in particular the underarm regions of the human body.

The method of controlling perspiration offered by use of the invention is particularly useful because the benefit can extend for a considerable period of time, sometimes greater than 24 hours. Significant deodorancy benefits may also accrue from the use of the present invention.

The fatty acid soap may be applied to the surface of the human body by any means. Liquid compositions comprising the fatty acid soap can be absorbed onto a carrier matrix like paper, fabric, or sponge and applied by contacting said carrier matrix with the surface.

The method of controlling perspiration is particularly useful when direct application to the surface of the human body is involved. This is especially true when application to the axillae and/or feet is involved. Use of the invention in the axillae is most beneficial because of the high concentration of eccrine sweat glands in these regions of the human body.

The method of controlling perspiration may involve multiple applications. It is thought that multiple applications improve perspiration reduction by some form of build-up effect. A composition comprising the fatty acid soap may be applied as part of one's daily regime, after washing, for example.

### The fatty acid soap

The C18-C22 fatty acid soap may be used to give antiperspirancy in the absence of any other agent capable of giving this benefit. Alternatively, the C18-C22 fatty acid soap may be used with another antiperspirant agent. Having said that, it is preferred that the C18-C22 fatty acid soap is used in the absence of any aluminium and/or zirconium salt. It is further preferred that the C18-C22 fatty acid soap is used from a single cosmetic composition.

When the C18-C22 fatty acid soap is used in conjunction with a further antiperspirant agent, it is not essential that the two are part of the same composition. Independent application of the two materials may be employed. Such application may be concurrent or consecutive, provided that the treated surface experiences the presence of both materials. When the actives are applied from independent compositions, it is preferred that the product also comprises a means for, and/or instruction for, both of the compositions to be applied to the surface requiring treatment.

When a further antiperspirant agent is employed, it preferably not an aluminium or zirconium salt.

The C18-C22 fatty acid soap is a soap of a saturated fatty acid, more preferably a soap of stearic or behenic acid and most preferably a soap of stearic acid. Sodium soaps are particularly preferred.

When the fatty acid soap is applied from a cosmetic composition, as it typically is, its level in the cosmetic composition is preferably from 2 to 50%, more preferably from 3 to 40% and most preferably from 4 to 25% of the total composition.

### Other Components

A highly preferred additional component used along with the fatty acid soap is a liquid carrier, particularly when the fatty acid soap is present as a dispersion in the liquid carrier.

Herein, components or features defined as having a particular state of matter, e.g. "liquid carriers" or "solid sticks" have that state of matter at 20°C and 1 atmosphere pressure.

When the fatty acid soap is present as a dispersion in a liquid carrier, the average particle size of the dispersion is typically from 1 to 100 microns and especially from 10 to 60 microns. Particle size distributions may be measured using light scattering techniques, such as laser light scattering.

In preferred embodiments, a mixture of liquid carriers is employed.

When employed, the total content of liquid carriers is preferably from 30 to 98%, more preferably from 50 to 95% and most preferably from 75 to 95%.

Preferred liquid carriers are glycols, such ethylene glycol, propylene glycol (PG) and dipropylene glycol (DPG). Particularly preferred glycols are PG and DPG.

Water may also be used as a liquid carrier, preferably in combination with a glycol and more preferably in combination with PG and/or DPG.

A particularly preferred liquid carrier mixture consists of water, PG and DPG, i.e. the carrier liquid consists solely of water, PG and DPG.

When both water and PG and/or DPG are employed, the ratio of PG and/or DPG to water is preferably at least 1: 1 and more preferably at least 2: 1. Interpedently and in conjunction with the above preferred ratios, it is preferred that the ratio of PG and/or DPG to water is preferably at most 40: 1 and more preferably at most 30: 1.

The preferred ratios described in the paragraph immediately above are particularly relevant when the carrier fluid consists of water, PG and DPG.

Without wishing to be bound by theory, it is believed that the presence of the preferred carriers and preferred carrier combinations, in dispersions of the fatty acid soap, give a phase structure for the dispersion that enhances the delivery and antiperspirancy performance of the fatty acid soap.

Glycols, in particular PG and/or DPG, are preferred components in the carrier fluid because they may serve to plasticize the fatty acid soap and enhance its film-formation, thereby enhancing its antiperspirant performance.

The carrier fluid may also comprise one or more oils.

Suitable oils include hydrocarbon oils and ester oils, particularly triglyceride oils, such as sunflower seed oil. Other particular oils that might be used are C₁₂₋₁₅ alkyl benzoate esters, hydrogenated polybutene, PPG-14 butyl ether, triethyl citrate, isopropyl palmitate, and isopropyl myristate.

When the fatty acid soap is used as a dispersion in a liquid carrier, is preferred that the dispersion takes the form of a solid stick. This can be possible without additional components because the fatty acid soap can serve as a structurant for the fatty acid soap dispersion. To achieve the form of a solid stick, the fatty acid soap preferably has a level of 5% or greater, this is particularly true soaps of palmitic acid or stearic acid and especially true for sodium stearate.

Other structurant may also be used in compositions suitable for use in accordance with the present invention. The total level of structurant, including the C18-C22 fatty acid soap, is preferably from 5 to 25%.

A preferred additional component in compositions used in the present invention is a dispersant. A dispersant is a component which serves to prevent or reduce agglomeration of a dispersion. The use of a dispersant together with the C18-C22 fatty acid soap, particularly a poloxamine, enhances the application and visual aesthetics of C18-C22 fatty acid soap dispersions.

When employed, the dispersant is used in the composition of which it is part at preferably from 0.1 to 10%, more preferably from 0.5 to 4% and most preferably from 1 to 3%. These preferences apply particularly when the dispersant is a poloxamine.

An anti-microbial deodorant agent is a preferred additional component of compositions suitable for use in accordance with the present invention. The anti-microbial deodorant agent may be a bacteriocidal agent or a bacteriostatic agent. Synergistic deodorancy benefits may be achieved using compositions comprising an anti-microbial deodorant agent.

Preferred anti-microbial deodorant agents are organic in nature and such anti-microbial deodorant agents are particularly preferred in compositions that do not comprise an astringent salt of aluminium and/or zirconium.

When employed, the anti-microbial deodorant agent is typically incorporated into the composition at from 0.01% to 3% and particularly at from 0.03% to 0.5%.

Preferred anti-microbial deodorant agents have a minimum inhibitory concentration (MIC) of 1 mg.ml⁻¹ or less, particularly 200 µg.ml⁻¹ or less, and especially 100 µg.ml⁻¹ or less.

The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ^{™} available from Zeneca PLC, preferably used at up to 1% and more preferably at 0.03% to 0.3%; 2',4,4'-trichloro,2-hydroxy-diphenyl ether (triclosan), preferably used at up to 1% of the composition and more preferably at 0.05-0.3%; and 3,7,11-trimethyldodeca-2,6,10-trienol (farnesol), preferably used at up to 1% and more preferably at up to 0.5%.

Other suitable organic antimicrobial agents are transition metal chelators, as described in WO01/52805, for example. Transitional metal chelators having a binding coefficient for iron(III) of greater than 10²⁶, for example diethylenetriaminepentaacetic acid and salts thereof are preferred.

Other cosmetically acceptable components may also be present in compositions used in accordance with the present invention. Such will vary according to the desired mode of application of the composition. For example, a volatile propellant is a typical component in compositions for spray application and an aqueous carrier fluid (usually water) is a typical component in compositions for roll-on application. Thickeners, in particular cellulosic thickeners such as hydroxypropylcellulose and hydroxyethylcellulose may also be used in roll-on compositions.

Certain sensory modifiers are further desirable components. Such materials are preferably used at a level of up to 20%. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely-divided silica (eg. Aerosil 200), particulate polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

Fragrance is also a desirable additional component. Suitable materials include conventional perfumes, such as perfume oils and also include so-called deo-perfumes, as described in EP 545,556 and other publications. Levels of incorporation are preferably up to 4%, particularly from 0.1% to 2%, and especially from 0.7% to 1.7%.

It should be noted that certain components of compositions perform more than one function. Such components are particularly preferred additional ingredients, their use often saving both money and formulation space. Examples of such components include ethanol, isopropyl myristate, and the many components that can act as sensory modifiers.

Further additional components that may also be included are colourants and preservatives at a conventional concentration, for example C₁-C₃ alkyl parabens.

The invention will now be illustrated by the following non-limiting examples.

### Examples

The following abbreviations are employed:
- DGP: dipropylene glycol
- PG: propylene glycol
- POE: poly(oxyethylene)
- PPG: poly(propylene glycol)
- SWR: Sweat Weight Reduction.

The stick compositions indicated in Tables 1 and 2 were prepared using methods known in the art.

The antiperspirancy performances of the compositions were compared in hot room clinical studies performed with 30 to 35 female volunteers. Following washing of the panellists' axillae, test operators applied ca. 0.30 g of each example to one axilla and a non-antiperspirant control (deodorant spray) to the other axilla of each panellist (randomised to avoid any left-right bias). This was done once each day for three days. After each application, panellists were requested not to wash under their arms for the following 24 hours.

24 hours after the third and final product application, the panellists were induced to sweat in a hot-room at 40°C (±2°C) and 40% (±5%) relative humidity, for 40 minutes. After this period, the panellists left the hot-room and their axillae were carefully wiped dry. Preweighed cotton pads were then applied to each axilla of each panellist and the panellists re-entered the hot-room for a further 20 minutes. Following this period, the pads were removed and re-weighed, enabling the weight of sweat generated to be calculated.

The relative sweat weight reduction (SWR) (Example vs. Control) for each panellist was calculated as a percentage (% SWR) and the mean % SWR was calculated according to the method described by Murphy and Levine in "Analysis of Antiperspirant Efficacy Results", J. Soc. Cosmetic Chemists, 1991 (May), 42, 167-197. The results are presented at the bottom of Tables 1 and 2.

**Table 1**

| Components | Examples | | | | |
|---|---|---|---|---|---|
| | 1 | 2 | 3 | 4 | 2 (repeat) |
| Na stearate | 11.00 | 5.50 | 11.00 | 11.00 | 5.50 |
| DPG | 58.96 | 40.40 | 9.02 | 8.02 | 40.40 |
| PG | 14.76 | 22.50 | 36.08 | 32.08 | 22.50 |
| Dispersant* | 1.00 | 3.00 | 1.00 | 6.00 | 3.00 |
| EDTA | 0.10 | 0.10 | 0.10 | 0.10 | 0.10 |
| BHT | 0.05 | 0.05 | 0.05 | 0.05 | 0.05 |
| Water | 14.13 | 28.45 | 42.75 | 42.75 | 28.45 |
| % SWR: | 30 | 24.3 | 16 | 17.5 | 27.4 |
| 95% levels | 23-36 | 15.0-32.6 | 8-24 | 8-25 | 17.9-35.8 |
| p-value | <0.01 | <0.01 | <0.01 | <0.01 | <0.01 |

| | | | | | |
|---|---|---|---|---|---|
| * Poloxamine 1307: POE/PPO block polymer of ethylene diamine with x = 23 (i.e. 23 oxypropylene units) and y = 74 (i.e. 74 oxyethylene units). | | | | | |

The SWR results given in Tales 1 and 2, in particular those for Examples 1, 2 and 5, are evidence of the surprisingly good antiperspirancy benefit found in accordance with the present invention.

**Table 2**

| Components | Examples | | | | |
|---|---|---|---|---|---|
| | 5 | 6 | 7 | 8 | 9 |
| Na stearate | 12.00 | 2.75 | 11.00 | 7.40 | 5.50 |
| DPG | 65.85 | 42.68 | 53.96 | 54.25 | 18.90 |
| PG | 17.00 | 10.67 | 14.74 | 30.20 | - |
| Dispersant* | 1.00 | 1.00 | 6.00 | 3.00 | 3.00 |
| EDTA | 0.10 | 0.10 | 0.10 | 0.10 | 0.10 |
| BHT | 0.05 | 0.05 | 0.05 | 0.05 | 0.05 |
| Water | 4.00 | 42.75 | 14.16 | 5.00 | 72.45 |
| % SWR: | 31 | 16 | 15 | 14 | 11 |
| 95% levels | 25-37 | 6-26 | 7-23 | 3-23 | 5-17 |
| p-value | <0.01 | <0.01 | <0.01 | <0.02 | <0.01 |

## Claims

1. The non-therapeutic use of a saturated C18-C22 fatty acid soap as an antiperspirant agent.

2. The use according to claim 1, wherein the fatty acid soap has 18 carbon atoms.

3. The use according to claim 2, wherein the fatty acid soap is sodium stearate

4. The use according to any of the preceding claims, wherein the C18-C22 fatty acid soap is dispersed in a liquid carrier and is topically applied to the skin of the human body.

5. The use according to claim 4, wherein the dispersion has the form of a solid stick.

6. The use according to claim 4 or claim 5, wherein the liquid carrier comprises propylene glycol and/or dipropylene glycol.

7. The use according to claim 6, wherein the liquid carrier comprises water.

8. The use according to claim 7, wherein the liquid carrier comprises propylene glycol and dipropylene glycol.

9. The use according to claim 8, wherein the liquid carrier wherein the liquid carrier consists of water, propylene glycol and dipropylene glycol.

10. The use according to any of claims 7 to 9, wherein the ratio of propylene glycol and/or dipropylene glycol to water is at least 1: 1 and preferably at least 2: 1 by weight.

11. The use according to any of claims 4 to 10, wherein the dispersion is free from any astringent aluminium or zirconium metal salt.

12. The use according to any of claims 4 to 11, wherein the C18-C22 fatty acid soap is present at from 4 to 25% by weight of the total dispersion.

13. The use according to any of claims 4 to12, wherein the dispersion comprises a poloxamine dispersant.

14. The use according to claim 13, wherein the poloxamine dispersant is present at from 0.5 to 4% by weight of the total dispersion.

## Patentansprüche

1. Nicht-therapeutische Verwendung einer gesättigten C18-C22-Fettsäureseife als Antitranspirant.

2. Verwendung nach Anspruch 1, wobei die Fettsäureseife 18 Kohlenstoffatome aufweist.

3. Verwendung nach Anspruch 2, wobei die Fettsäureseife Natriumstearat ist.

4. Verwendung nach irgendeinem der vorhergehenden Ansprüche, wobei die C18-C22-Fettsäureseife in einem flüssigen Träger dispergiert ist und topisch auf die Haut des menschlichen Körpers aufgetragen wird.

5. Verwendung nach Anspruch 4, wobei die Dispersion die Form eines festen Stifts aufweist.

6. Verwendung nach Anspruch 4 oder 5, wobei der flüssige Träger Propylenglycol und/oder Dipropylenglycol umfasst.

7. Verwendung nach Anspruch 6, wobei der flüssige Träger Wasser umfasst.

8. Verwendung nach Anspruch 7, wobei der flüssige Träger Polypropylenglycol und Dipropylenglycol umfasst.

9. Verwendung nach Anspruch 8, wobei der flüssige Träger aus Wasser, Propylenglycol und Dipropylenglycol besteht.

10. Verwendung nach einem der Ansprüche 7 bis 9, wobei das Verhältnis von Propylenglycol und/oder Dipropylenglycol zu Wasser mindestens 1:1 und vorzugsweise mindestens 2:1, bezogen auf das Gewicht, beträgt.

11. Verwendung nach irgendeinem der Ansprüche 4 bis 10, wobei die Dispersion frei von jeglichen astringenten Aluminium- oder Zirconiummetallsalzen ist.

12. Verwendung nach einem der Ansprüche 4 bis 11, wobei die C18-C22-Fettsäureseife mit 4 bis 25 Gewichts-% der gesamten Dispersion vorliegt.

13. Verwendung nach einem der Ansprüche 4 bis 12, wobei die Dispersion ein Poloxamin-Dispergiermittel umfasst.

14. Verwendung nach Anspruch 13, wobei das Poloxamin-Dispergiermittel mit 0,5 bis 4 Gewichts-% der gesamten Dispersion vorliegt.

## Revendications

1. Utilisation non-thérapeutique d'un savon d'acide gras en C18-C22 saturé comme un agent antiperspirant.

2. Utilisation selon la revendication 1, dans laquelle le savon d'acide gras présente 18 atomes de carbone.

3. Utilisation selon la revendication 2, dans laquelle le savon d'acide gras est le stéarate de sodium.

4. Utilisation selon l'une quelconque des revendications précédentes, dans laquelle le savon d'acide gras en C18-C22 est dispersé dans un support liquide et est topiquement appliqué à la peau du corps humain.

5. Utilisation selon la revendication 4, dans laquelle la dispersion présente la forme d'un stick solide.

6. Utilisation selon la revendication 4 ou revendication 5, dans laquelle le support liquide comprend du propylène glycol et/ou dipropylène glycol.

7. Utilisation selon la revendication 6, dans laquelle le support liquide comprend de l'eau.

8. Utilisation selon la revendication 7, dans laquelle le support liquide comprend du propylène glycol et dipropylène glycol.

9. Utilisation selon la revendication 8, dans laquelle le support liquide consiste en eau, propylène glycol et dipropylène glycol.

10. Utilisation selon l'une quelconque des revendications 7 à 9, dans laquelle le rapport de propylène glycol et/ou dipropylène glycol à eau est d'au moins 1:1 et de préférence d'au moins 2:1 en masse.

11. Utilisation selon l'une quelconque des revendications 4 à 10, dans laquelle la dispersion est exempte de tout sel de métal d'aluminium ou zirconium astringent.

12. Utilisation selon l'une quelconque des revendications 4 à 11, dans laquelle le savon d'acide gras en C18-C22 est présent à de 4 à 25 % en masse de la dispersion totale.

13. Utilisation selon l'une quelconque des revendications 4 à 12, dans laquelle la dispersion comprend un dispersant de poloxamine.

14. Utilisation selon la revendication 13, dans laquelle le dispersant de poloxamine est présent à de 0,5 à 4 % en masse de la dispersion totale.
